# EUROPEAN PATENT SPECIFICATION

(11) **EP 1 257 555 B1**
(45) Date of publication and mention of the grant of the patent: **03.12.2003**
(21) Application number: 01904731.5
(22) Date of filing: 07.02.2001
(51) Int. Cl.: C07D 513/04, C07D 487/04, A61K 31/519, A61P 11/00, A61P 17/06, A61P 29/00

(54) **PYRIMIDINE COMPOUNDS AND THEIR USE AS MODULATORS OF CHEMOKINE RECEPTOR ACTIVITY**
PYRIMIDINVERBINDUNGEN UND IHRE VERWENDUNG ALS MODULATOREN DER CHEMOKIN-REZEPTOR-AKTIVITÄT
COMPOSES PYRIMIDINIQUES ET LEUR UTILISATION COMME MODULATEURS DE L'ACTIVITE DES RECEPTEURS DE CHIMIOKINE

(30) Priority: 11.02.2000 GB 0003022; 11.02.2000 GB 0003023
(43) Date of publication of application: 20.11.2002
(73) Proprietor: AstraZeneca AB, 151 85 Södertälje (SE)
(72) Inventor: BONNERT, Roger, AstraZeneca, Loughborough, Leics LE11 5RH (GB); HUNT, Fraser, AstraZeneca, Loughborough, Leics LE11 5RH (GB); WILLIS, Paul, AstraZeneca, Loughborough, Leics LE11 5RH (GB)
(74) Representative: Bryant, Tracey
(86) International application number: SE0100246
(87) International publication number: WO01058906

(56) References cited:
- EP-A1- 0 778 277
- WO-A1-00/09511
- WO-A1-98/08847
- WO-A1-98/25617
- WO-A1-99/51608
- DE-A1- 2 331 223

## Description

The present invention relates to certain pyrimidine compounds, processes and intermediates used in their preparation, pharmaceutical compositions containing them and their use in therapy.

Chemokines play an important role in immune and inflammatory responses in various diseases and disorders, including asthma and allergic diseases, as well as autoimmune pathologies such as rheumatoid arthritis and atherosclerosis. These small secreted molecules are a growing superfamily of 8-14 kDa proteins characterised by a conserved four cysteine motif. At the present time, the chemokine superfamily comprises three groups exhibiting characteristic structural motifs, the C-X-C, C-C and C-X₃-C families. The C-X-C and C-C families have sequence similarity and are distinguished from one another on the basis of a single amino acid insertion between the NH-proximal pair of cysteine residues. The C-X₃-C family is distinguished from the other two families on the basis of having a triple amino acid insertion between the NH-proximal pair of cysteine residues.

The C-X-C chemokines include several potent chemoattractants and activators of neutrophils such as interleukin-8 (IL-8) and neutrophil-activating peptide 2 (NAP-2).

The C-C chemokines include potent chemoattractants of monocytes and lymphocytes but not neutrophils. Examples include human monocyte chemotactic proteins 1-3 (MCP-1, MCP-2 and MCP-3), RANTES (Regulated on Activation, Normal T Expressed and Secreted), eotaxin and the macrophage inflammatory proteins 1α and 1β (MIP-1α and MIP-1β).

The C-X₃-C chemokine (also known as fractalkine) is a potent chemoattractant and activator of microglia in the central nervous system (CNS) as well as of monocytes, T cells, NK cells and mast cells.

Studies have demonstrated that the actions of the chemokines are mediated by subfamilies of G protein-coupled receptors, among which are the receptors designated CCR1, CCR2, CCR2A, CCR2B, CCR3, CCR4, CCR5, CCR6, CCR7, CCR8, CCR9, CCR10 and CCR11 (for the C-C family); CXCR1, CXCR2, CXCR3, CXCR4 and CXCR5 (for the C-X-C family) and CX₃CR1 for the C-X₃-C family. These receptors represent good targets for drug development since agents which modulate these receptors would be useful in the treatment of disorders and diseases such as those mentioned above.

In accordance with the present invention, there is therefore provided compounds of formula (I) or pharmaceutically acceptable salts or solvates thereof: 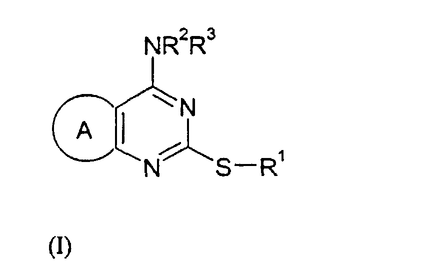 in which:
A is a group of formula (a), (b) or (c): 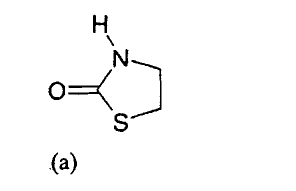 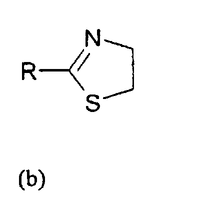 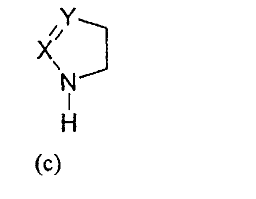 in which:
   R¹ represents an optionally branched C₁-C₄ alkyl group which terminates in an aryl or heteroaryl group each of which can be optionally substituted by one or more substituents independently selected from halogen atoms, cyano, nitro, -OR⁴, -NR⁵R⁶, -CONR⁵R⁶, -COOR⁷, -NR⁸COR⁹, -SR⁹, -SO₂R⁹, -SO₂NR⁵R⁶, -NR⁸SO₂R⁹, C₁-C₆ alkyl or trifluoromethyl groups and which may optionally contain one or more atoms independently selected from, O, NR⁵, or S.
   R² represents an optionally branched C₁-C₈ alkyl group terminating in an OH substituent which may be optionally substituted by one or more substituent groups independently selected from -OR⁴, -NR⁵R⁶ -CONR⁵R⁶, -NR⁸COR⁹, -SR⁹, -SO₂R⁹, -SO₂NR⁵R⁶, - NR⁸SO₂R⁹,
   R³ represents hydrogen or C₁-C₆ alkyl optionally substituted by one or more substituent groups independently selected from halogen atoms, phenyl, -OR¹⁰ and -NR¹¹R¹²,
   R⁴ represents hydrogen, C₁-C₆ alkyl or a phenyl group the latter two of which may be optionally substituted by one or more substituent groups independently selected from halogen atoms, phenyl, -OR¹⁰ and -NR⁵R⁶.
   R⁵ and R⁶ independently represent a hydrogen atom or a C₁-C₆ alkyl or phenyl group the latter two of which may be optionally substituted by one or more substituent groups independently selected from halogen atoms, phenyl, -OR¹³ and -NR¹⁴R¹⁵, -CONR¹⁴R¹⁵, -NR¹⁴COR¹⁵, -SO₂NR¹⁴R¹⁵, NR¹⁴SO₂R¹⁵
      or
   R⁵ and R⁶ together with the nitrogen atom to which they are attached form a 4- to 7-membered saturated heterocyclic ring system optionally comprising a further heteroatom selected from oxygen and nitrogen atoms, which ring system may be optionally substituted by one or more substituent groups independently selected from phenyl, -OR¹³, -COOR¹³, -NR¹⁴R¹⁵, -CONR¹⁴R¹⁵, -NR¹⁴COR¹⁵, -SO₂NR¹⁴R¹⁵, -NR¹⁴SO₂R¹⁵ or C₁-C₆ alkyl, itself optionally substituted by one or more substituents independently selected from halogen atoms and -NR¹⁴R¹⁵ and -OR¹⁶ groups,
   X represents a nitrogen atom or CH ;
   Y represents a nitrogen atom or CH;
   R represents a hydrogen atom, or a group -NR¹⁷R¹⁸;
   R¹⁷ and R¹⁸ each independently represent a hydrogen atom, or a 4-piperidinyl, C₃-C₆ cycloalkyl or C₁-C₈ alkyl group, which latter two groups may be optionally substituted by one or more substituent groups independently selected from halogen atoms and -NR⁵R⁶,-CONR⁵R⁶, -OR⁹, -COOR⁷, -NR⁸COR⁹, -SR¹¹, -SO₂R¹¹, -SO₂NR⁵R⁶, -NR⁸SO₂R⁹, morpholinyl, C₁-C₄ alkyl, C₃-C₆ cycloalkyl, tetrahydrofuranyl, aryl and heteroaryl groups, each of the latter two groups may be optionally substituted by one or more substituents independently selected from halogen atoms, cyano, nitro, -NR⁵R⁶, -CONR⁵R⁶, -OR⁹, -NR⁸COR⁹, -SO₂NR⁵R⁶, -NR⁸SO₂R⁹, C₁-C₆ alkyl and trifluoromethyl groups
      where R⁵, R⁶, R⁸ and R⁹ are as defined,
      or R¹⁷ and R¹⁸ together with the nitrogen atom to which they are attached form a 4- to 7-membered saturated heterocyclic ring system, which ring system may be optionally substituted by one or more substituent groups independently selected from 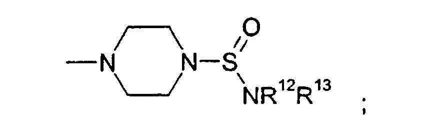 -NR⁵R⁶, -CONR⁵R⁶, -OR⁹, -COOR⁷, -NR⁸COR⁹, and C₁-C₆ alkyl optionally substituted by one or more substituents independently selected from halogen atoms and -NR¹²R¹³ and -OR⁸ groups,
   R⁹ represents a hydrogen atom, a C₁-C₆ alkyl group or a phenyl group, each of which may be optionally substituted by one or more substituent groups independently selected from halogen atoms, phenyl, -OR¹⁶ and -NR¹⁴R¹⁵, and
   each of R⁷, R⁸, R¹⁰, R¹¹, R¹², R¹³, R¹⁴ R¹⁵, and R¹⁶ independently represents a hydrogen atom or a C₁-C₆, alkyl, or a phenyl group,
      - provided that when Y is CH, then X is N.

In the context of the present specification, unless otherwise indicated, an alkyl or alkenyl group or an alkyl or alkenyl moiety in a substituent group may be linear or branched.

Aryl groups include phenyl and naphthyl. Heteroaryl is defined herein as a 5- or 6-membered aromatic ring optionally containing one or more heteroatoms selected from N, S and O. Examples include pyridine, pyrimidine, thiazole, oxazole, isoxazole, pyrazole, imidazole, furan and thiophene. Heterocyclic rings as defined for R¹⁷ and R¹⁸ means saturated heterocycles, examples of which include morpholine, azetidine, pyrrolidine, piperidine and piperazine.

Certain compounds of formula (I) are capable of existing in stereoisomeric forms. It will be understood that the invention encompasses all geometric and optical isomers of the compounds of formula (I) and mixtures thereof including racemates. Tautomers and mixtures thereof also form an aspect of the present invention.

In compounds of formula (I) where A is a group (b), the group R represents a hydrogen atom, or a group NR¹⁷R¹⁸. Particularly advantageous compounds of formula (I) are those in which R represents a group -NR¹⁷R¹⁸, in particular those in which R¹⁷ and R¹⁸ each independently represent a hydrogen atom, or a C₁-C₆ alkyl group substituted by a -CONR⁵R⁶ or imidazolyl (e.g. 1*H*-imidazol-4-yl) group.

In formula (I) above, the group R¹ represents an optionally branched C₁-C₄ alkyl group which terminates in an aryl or heteroaryl group each of which can be optionally substituted by one or more substituents independently selected from halogen atoms, cyano, nitro,-OR⁴, -NR⁵R⁶, -CONR⁵R⁶, -COOR⁷, -NR⁸COR⁹, -SR⁹, -SO₂R⁹, -SO₂NR⁵R⁶, -NR⁸SO₂R⁹, C₁-C₆ alkyl or trifluoromethyl groups and which may optionally contain one or more atoms independently selected from, O, NR⁵, or S. Particularly advantageous compounds of formula (I) are those in which R¹ represents an optionally substituted benzyl group. More preferably R¹ represents benzyl or benzyl substituted by one or more halogen atoms, in particular benzyl substituted by two fluoro atoms.

Suitably R² represents an optionally branched C₁-C₈ alkyl group terminating in an OH substituent which may be optionally substituted by one or more substituent groups independently selected from -OR⁴, -NR⁵R⁶ -CONR⁵R⁶, -NR⁸COR⁹, -SR⁹, -SO₂R⁹, -SO₂NR⁵R⁶, -NR⁸SO₂R⁹.

Preferably R² is CH(CH₃)CH₂OH, CH(Et)CH₂OH or C(CH₃)₂CH₂OH. When R² is CH(CH₃)CH₂OH or CH(Et)CH₂OH the resulting compounds of formula (I) are preferably in the form of the (R) isomer. Most preferably R² is CH(CH₃)CH₂OH.

Suitably R³ represents hydrogen or C₁-C₆ alkyl optionally substituted by one or more substituent groups independently selected from halogen atoms, phenyl, -OR¹⁰ and - NR¹¹R¹². Preferably R³ represents hydrogen.

Particularly preferred compounds of the invention include:
(2*R*)-2-[[2-Amino-5-[(phenylmethyl)thio]thiazolo[5,4-*d*]pyrimidin-7-yl]amino]-1-propanol.
(1*R*)-5-[[Phenylmethyl]thio]-7-[(2-hydroxy-1-methylethyl)amino]thiazolo[5,4-*d*]pyrimidin-2(1*H*)-one.
(2*R*)-2-[2-[(phenylmethyl)thio]-(1*H*-purin-6-yl)amino]-1-propanol
(2*R*)-2-[[6-[[(2,3-Difluorophenyl)methyl]thio]-1*H*-pyrazolo[3,4-*d*]pyrimidin-4-yl]amino]-1-propanol and
2-Methyl-2-[[5-[(phenylmethyl)thio]-3*H*-[1,2,3]-triazolo[4,5-*d*]pyrimidin-7-yl]amino]-1-propanol
and their pharmaceutically acceptable salts and solvates.

According to the invention there is also provided a process for the preparation of compounds of formula (I) which comprises:
(1) for compounds of formula (I) in which A is a group (a), treatment of a compound of formula (II): 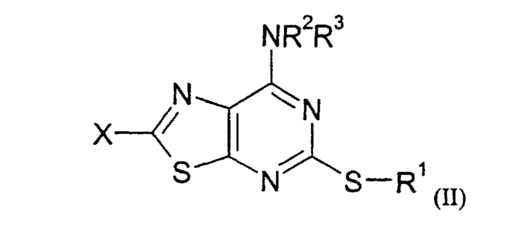 where R¹, R² and R³ are as defined in formula (I) or are protected derivatives thereof and X is a leaving group with aqueous acid, or
(2) for compounds of formula (I) in which A is a group of formula (c), treatment of a compound of formula (IIA): 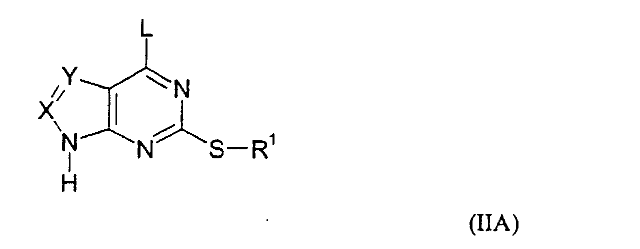 where X, Y and R¹ are as defined in formula (I) or are protected derivatives thereof and L is a leaving group with an amine HNR²R³, or
(3) for compounds of formula (I) in which A is a group of formula (c) and X and Y are both N, reacting a compound of general formula (IIB): 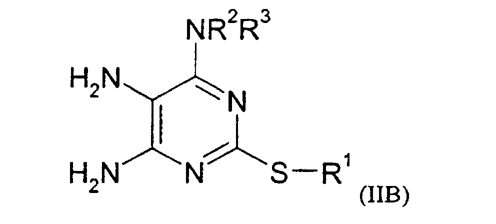 wherein R¹, R² and R³ are as defined in formula (I) or are protected derivatives thereof with a nitrosating agent, or
(4) for compounds of formula (I) A is a group (b) and where R¹, R² and R³ are as defined in formula (I) or are protected derivatives thereof and R is NH₂, treating a compound of formula (III) with a reducing agent: 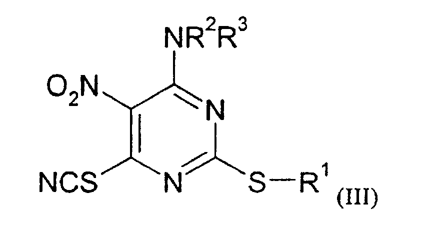 and optionally thereafter any of processes 1 to 4:
   - removing any protecting groups present
   - forming a pharmaceutically acceptable salt or solvate.

For process 1, suitable leaving groups X include alkoxyl groups such as methoxyl. The reaction may be carried out in a solvent such as dioxan using hydrochloric acid at a temperature between 0°C and 100°C.

Compounds of formula (II) where R¹, R² and R³ are as defined in formula (I) and X is a leaving group such as methoxyl may be prepared from the corresponding compounds (II) where R¹, R² and R³ are as defined above and X is a halogen such as bromine by treatment with a metal alkoxide. The reaction may be carried out using potassium hydroxide in methanol at room temperature.

Compounds of formula (II) where R¹, R² and R³ are as defined in formula (I) and X is a halogen may be prepared from compounds of formula (I) where A is (b) and where R¹, R² and R³ are as defined above and R is NH₂ by treatment with with a diazotizing agent such as *tert*-butyl nitrite and a halogenating agent such as bromoform.

Compounds of formula (I) where A is (b) and where R¹, R² and R³ are as defined in formula (I) and R is NH₂ may be prepared by treating a compound of formula (III) with a reducing agent such as iron powder. The reaction may be carried out in the presence of ammonium chloride in aqueous ethanol at reflux.

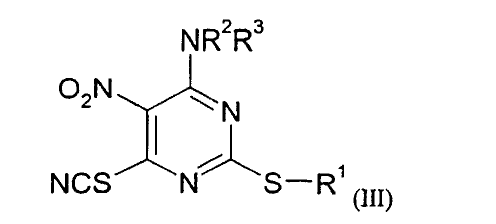

Compounds of formula (III) where R¹, R² and R³ are as defined in formula (I) may be prepared by treating a compound of formula (IV) where R¹, R² and R³ are as defined above and L is a leaving group such as halogen (e.g. chlorine) with a metal thiocyanate. The reaction may be carried out using potassium thiocyanate in dimethylformamide at room temperature.

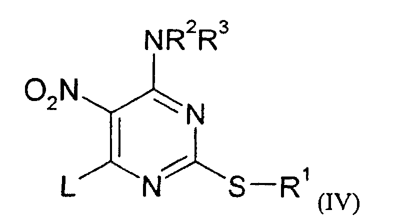

Compounds of formula (IV) where R¹, R² and R³ are as defined in formula (I) and L is a leaving group such as halogen (e.g. chlorine) may be prepared by treating a compound of formula (V) where R¹, R², R³ and L are as defined above with an amine HNR²R³. The reaction may be carried out in the presence of a base such as diisopropylethylamine in a solvent such as tetrahydrofuran at a temperature between 0 and 100°C.

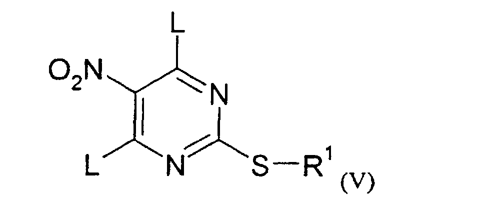

Compounds of formula (V) where R¹ is as defined in formula (I) and L is a halogen may be prepared by treating a compound of formula (V) where R¹ is as defined in formula (I) and L is an hydroxyl group with a halogenating agent such as phosphorus oxychloride. The reaction may be carried out in a solvent such as toluene at 100°C.

Compounds of formula (V) where R¹ is as defined in formula (I) and L is an hydroxyl group may be prepared by treating a compound of formula (VI) where R¹ is as defined in formula (I) and L is an hydroxyl group with a nitrating agent such as concentrated nitric acid. The reaction may be carried out in a solvent such as glacial acetic acid at a temperature between 0°C and 100°C.

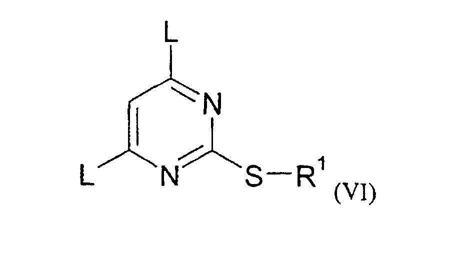

Compounds of formula (VI) where R¹ is as defined in formula (I) and L is an hydroxyl group are suitably prepared by reacting a compound of formula (VII): 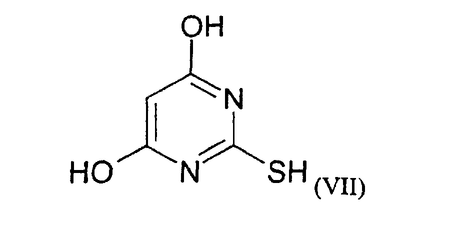 with a compound of formula R¹X where R¹ is as defined above and X is a leaving group such as bromide in the presence of a base such as sodium hydroxide. The reaction may be carried out in aqueous NMP at room temperature.

The reaction of compounds of formula (IIA) with an amine HNR²R³ can be carried out in a solvent such as N-methyl-pyrrolidinone at a temperature between 0°C and 150°C. Suitable leaving groups L include halogen, especially chloro.

The reaction of compounds of formula (IIB) with a nitrosating agent can be carried out in a solvent such as acetonitrile at reflux. Suitable nitrosating agents include isoamyl nitrite.

Compounds of formula (IIA) where L is halogen can be prepared by treating a compound of formula (IIA) where X, Y and R¹ are as defined in formula (I) and L is an hydroxyl group with a halogenating agent such as phosphorus oxychloride. The reaction may be carried out at reflux in the presence of dimethylaniline.

Compounds of formula (IIA) where L is hydroxyl group and X,Y and R¹ are defined in formula (I) are suitably prepared by reacting a compound of formula (VIII): 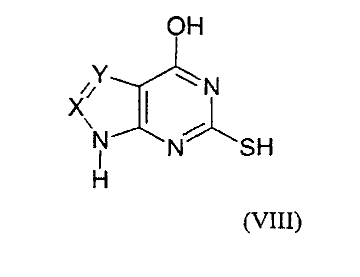 with a compound of formula R¹L¹ where R¹ is as defined above and L¹ is a leaving group such as bromide in the presence of a base such as sodium hydroxide. The reaction may be carried out in aqueous ethanol at room temperature.

Compounds of formula (IIB) where R¹, R² and R³ are as defined in formula (I) are suitably prepared by reacting a compound of formula (IX) with a reducing agent such as sodium hydrosulphite. The reaction may be carried out in aqueous dioxane at a temperature between 20°C and 100°C.

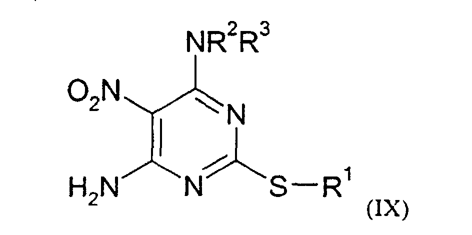

Compounds of formula (IX) where R¹, R² and R³ are as defined in formula (I) are suitably prepared by reacting a compound of formula (X): 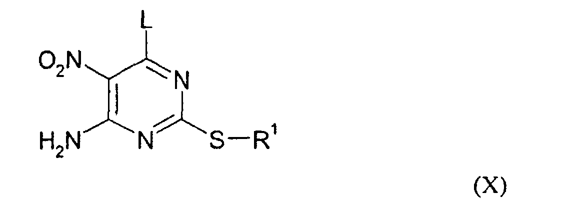 wherein L represents a leaving group such as a halogen atom (e.g. chlorine) and R¹ is as defined in formula (I) with an amine HNR²R³. The reaction may be carried out in a solvent such as dimethylformamide at a temperature between 0°C and 150°C.

Compounds of formula (X) where R¹ is as defined in formula (I) are suitably prepared by reacting compounds of formula (V) where R¹ is as defined in formula (I) with ammonia. The reaction may be carried out in a solvent such as diethyl ether at room temperature.

Compounds of formula (VII) and formula (VIII) are either commercially available or can be prepared using standard chemistry.

It will be appreciated by those skilled in the art that in the processes of the present invention certain functional groups such as hydroxyl or amino groups in the starting reagents or intermediate compounds may need to be protected by protecting groups. Thus, the preparation of the compounds of formula (I) may involve, at an appropriate stage, the removal of one or more protecting groups.

The protection and deprotection of functional groups is fully described in 'Protective Groups in Organic Chemistry', edited by J. W. F. McOmie, Plenum Press (1973), and 'Protective Groups in Organic Synthesis', 2nd edition, T. W. Greene & P. G. M. Wuts, Wiley―Interscience (1991).

Novel intermediate compounds form a further aspect of the invention.

The compounds of formula (I) above may be converted to a pharmaceutically acceptable salt or solvate thereof, preferably a basic addition salt such as sodium, potassium, calcium, aluminium, lithium, magnesium, zinc, benzathine, chloroprocaine, choline, diethanolamine, ethanolamine, ethyldiamine, meglumine, tromethamine or procaine, or an acid addition salt such as a hydrochloride, hydrobromide, phosphate, acetate, fumarate, maleate, tartrate, citrate, oxalate, methanesulphonate or *p*-toluenesulphonate.

The compounds of formula (I) have activity as pharmaceuticals, in particular as modulators of chemokine receptor (especially CXCR2) activity, and may be used in the treatment (therapeutic or prophylactic) of conditions/diseases in human and non-human animals which are exacerbated or caused by excessive or unregulated production of chemokines. Examples of such conditions/diseases include:
(1) **(the respiratory tract)** obstructive airways diseases including chronic obstructive pulmonary disease (COPD); asthma, such as bronchial, allergic, intrinsic, extrinsic and dust asthma, particularly chronic or inveterate asthma (e.g. late asthma and airways hyper-responsiveness); bronchitis; acute, allergic, atrophic rhinitis and chronic rhinitis including rhinitis caseosa, hypertrophic rhinitis, rhinitis purulenta, rhinitis sicca and rhinitis medicamentosa; membranous rhinitis including croupous, fibrinous and pseudomembranous rhinitis and scrofoulous rhinitis; seasonal rhinitis including rhinitis nervosa (hay fever) and vasomotor rhinitis; sarcoidosis, farmer's lung and related diseases, fibroid lung and idiopathic interstitial pneumonia;
(2) **(bone and joints)** rheumatoid arthritis, seronegative spondyloarthropathies (including ankylosing spondylitis, psoriatic arthritis and Reiter's disease), Behcet's disease, Sjogren's syndrome and systemic sclerosis;
(3) (**skin**) psoriasis, atopical dermatitis, contact dermatitis and other eczmatous dermitides, seborrhoetic dermatitis, Lichen planus, Pemphigus, bullous Pemphigus, Epidermolysis bullosa, urticaria, angiodermas, vasculitides, erythemas, cutaneous eosinophilias, uveitis, Alopecia areata and vernal conjunctivitis;
(4) **(gastrointestinal tract)** Coeliac disease, proctitis, eosinopilic gastro-enteritis, mastocytosis, Crohn's disease, ulcerative colitis, food-related allergies which have effects remote from the gut, e.g., migraine, rhinitis and eczema;
(5) (**central and peripheral nervous system**) Neurodegenerative diseases and dementia disorders, e.g. Alzheimer's disease, amyotrophic lateral sclerosis and other motor neuron diseases, Creutzfeldt-Jacob's disease and other prion diseases, HIV encephalopathy (AIDS dementia complex), Huntington's disease, frontotemporal dementia, Lewy body dementia and vascular dementia; polyneuropathies, e.g. Guillain-Barré syndrome, chronic inflammatory demyelinating polyradiculoneuropathy, multifocal motor neuropathy, plexopathies; CNS demyelination, e.g. multiple sclerosis, acute disseminated/haemorrhagic encephalomyelitis, and subacute sclerosing panencephalitis; neuromuscular disorders, e.g. myasthenia gravis and Lambert-Eaton syndrome; spinal diorders, e.g. tropical spastic paraparesis, and stiff-man syndrome: paraneoplastic syndromes, e.g. cerebellar degeneration and encephalomyelitis; CNS trauma; migraine; and stroke.
(6) (**other tissues and systemic disease**) atherosclerosis, Acquired Immunodeficiency Syndrome (AIDS), lupus erythematosus, systemic lupus, erythematosus, Hashimoto's thyroiditis, type I diabetes, nephrotic syndrome, eosinophilia fasc tis, hyper IgE syndrome, lepromatous leprosy, and idiopathic thrombocytopenia pupura; post-operative adhesions, and sepsis.
(7) **(allograft rejection)** acute and chronic following, for example, transplantation of kidney, heart, liver, lung, bone marrow, skin and cornea; and chronic graft versus host disease;
(8) Cancers, especially non-small cell lung cancer (NSCLC), malignant melanoma, prostate cancer and squamous sarcoma, and tumour metastasis;
(9) Diseases in which angiogenesis is associated with raised CXCR2 chemokine levels (e.g. NSCLC, diabetic retinopathy).
(10)Cystic fibrosis, re-perfusion injury in the heart, brain, peripheral limbs and other organs.
(11) Burn wounds & chronic skin ulcers
(12) Reproductive Diseases (e.g. Disorders of ovulation, menstruation and implantation, Pre-term labour, Endometriosis)

Thus, the present invention provides a compound of formula (I), or a pharmaceutically-acceptable salt or solvate thereof, as hereinbefore defined for use in therapy.

Preferably the compounds of the invention are used to treat diseases in which the chemokine receptor belongs to the CXC chemokine receptor subfamily, more preferably the target chemokine receptor is the CXCR2 receptor.

Particular conditions which can be treated with the compounds of the invention are psoriasis, diseases in which angiogenesis is associated with raised CXCR2 chemokine levels, and COPD. It is preferred that the compounds of the invention are used to treat psoriasis.

As a further aspect of the present invention, certain compounds of formula (I) may have utility as antagonists of the CX3CR1 receptor. Such compounds are expected to be particularly useful in the treatment of disorders within the central and peripheral nervous system and other conditions characterized by an activation of microglia and/or infiltration of leukocytes (e.g. stroke/ischemia and head trauma).

In a further aspect, the present invention provides the use of a compound of formula (I), or a pharmaceutically acceptable salt or solvate thereof, as hereinbefore defined in the manufacture of a medicament for use in therapy.

In a still further aspect, the present invention provides the use of a compound of formula (I), or a pharmaceutically acceptable salt or solvate thereof, as hereinbefore defined in the manufacture of a medicament for the treatment of human diseases or conditions in which modulation of chemokine receptor activity is beneficial, in particular modulation of the CXCR2 receptor.

In the context of the present specification, the term "therapy" also includes "prophylaxis" unless there are specific indications to the contrary. The terms "therapeutic" and "therapeutically" should be construed accordingly.

The invention still further provides a method of treating a chemokine mediated disease wherein the chemokine binds to a chemokine (especially CXCR2) receptor, which comprises administering to a patient a therapeutically effective amount of a compound of formula (I), or a pharmaceutically acceptable salt or solvate thereof, as hereinbefore defined.

The invention also provides a method of treating an inflammatory disease, especially psoriasis, in a patient suffering from, or at risk of, said disease, which comprises administering to the patient a therapeutically effective amount of a compound of formula (I), or a pharmaceutically acceptable salt or solvate thereof, as hereinbefore defined.

For the above-mentioned therapeutic uses the dosage administered will, of course, vary with the compound employed, the mode of administration, the treatment desired and the disorder indicated.

The compounds of formula (I) and pharmaceutically acceptable salts and solvates thereof may be used on their own but will generally be administered in the form of a pharmaceutical composition in which the formula (I) compound/salt/solvate (active ingredient) is in association with a pharmaceutically acceptable adjuvant, diluent or carrier.

Depending on the mode of administration, the pharmaceutical composition will preferably comprise from 0.05 to 99 %w (per cent by weight), more preferably from 0.05 to 80 %w, still more preferably from 0.10 to 70 %w, and even more preferably from 0.10 to 50 %w, of active ingredient, all percentages by weight being based on total composition.

The present invention also provides a pharmaceutical composition comprising a compound of formula (I), or a pharmaceutically acceptable salt or solvate thereof, as hereinbefore defined, in association with a pharmaceutically acceptable adjuvant, diluent or carrier.

The invention further provides a process for the preparation of a pharmaceutical composition of the invention which comprises mixing a compound of formula (I), or a pharmaceutically acceptable salt or solvate thereof, as hereinbefore defined, with a pharmaceutically acceptable adjuvant, diluent or carrier.

The pharmaceutical compositions may be administered topically (e.g. to the lung and/or airways or to the skin) in the form of solutions, suspensions, heptafluoroalkane aerosols and dry powder formulations; or systemically, e.g. by oral administration in the form of tablets, capsules, syrups, powders or granules, or by parenteral administration in the form of solutions or suspensions, or by subcutaneous administration or by rectal administration in the form of suppositories or transdermally. Preferably the compounds of the invention are administered orally.

The invention will now be further illustrated by reference to the following examples. In the examples the Nuclear Magnetic Resonance (NMR) spectra were measured on a Varian Unity Inova 300 or 400 MHz spectrometer and the Mass Spectrometry (MS) spectra measured on a Finnigan Mat SSQ7000 or Micromass Platform spectrometer. Where necessary, the reactions were performed under an inert atmosphere of either nitrogen or argon. Chromatography was generally performed using Matrex Silica 60® (35-70 micron) or Prolabo Silica gel 60® (35-70 micron) suitable for flash silica gel chromatography. High pressure liquid chromatography purification was performed using either a Waters Micromass LCZ with a Waters 600 pump controller, Waters 2487 detector and Gilson FC024 fraction collector or a Waters Delta Prep 4000. The abbreviations m.p. and DMSO used in the examples stand for melting point and dimethyl sulphoxide respectively.

### Example 1

### (2R)-2-[[2-Amino-5-[(phenylmethyl)thio]thiazolo[5,4-d]pyrimidin-7-yl]amino]-1-propanol.

### a) 2-[(Phenylmethyl)thio]-4,6(1H,5H)-pyrimidinedione

To a solution of 4,6-dihydroxy-2-mercaptopyrimidine (100 g) in 5M NaOH (360 ml) was added first NMP (200 ml), then benzyl bromide (90 ml) dropwise over 2 hours. The reaction was stirred at room temperature for 24 hours, then acidified to pH 2 with cone HCl (100 ml) added dropwise over 2 hours at -5°C to give a pink precipitate. This was isolated by decanting off the solution followed by trituration with diethyl ether (1 litre) to give the subtitled product as a white powder after filtration and drying (210 g).
m.p. 220-250°C (dec)
MS: APCI (-ve) 233 (M-H)
¹H NMR: δ (DMSO) 11.76 (1H, br s), 7.45 (2H, d), 7.26 (3H, m), 5.18 (1H, s), 4.38 (2H, s).

### b) 4,6-Dihydroxy-5-nitro-2-[(phenylmethyl)thio]pyrimidine

The product of step (a) (2 g) was added to a mixture of glacial acetic acid (50 ml) and concentrated nitric acid (20 ml) and the reaction mixture heated to 50°C. A further 28 g of the product of step (a) was added in portions over 2 hours whilst maintaining the reaction temperature between 50 and 60°C. After stirring the reaction mixture for a further 1 hour at 50°C it was poured onto crushed ice and the subtitled product isolated by filtration as a yellow solid (12.3 g).
¹H NMR: δ (DMSO) 7.48-7.19 (5H, m), 4.47 (2H, s).

### c) 4,6-Dichloro-5-nitro-2-[(phenylmethyl)thio]-pyrimidine

A suspension of the product of step (b) ( 59.2 g) in a mixture of POCl₃ (100 ml) and toluene (400 ml) was heated to 80°C. A solution of 1-methylimidazole (16.9 ml) in toluene (200 ml) was added dropwise over 1 hour and the reaction mixture then heated at 100°C for 24 hours. After cooling to room temperature, the solvent was removed by evaporation and water (2 litres) cautiously added to the residue. The mixture was extracted with dichloromethane (4x500 ml) and the combined organic extracts dried over MgSO₄, filtered and evaporated to give a brown tar. This was purified by column chromatography, eluting with 10% dichloromethane in isohexane, to afford the subtitled product as a yellow solid (29.7 g).
MS: APCI (-ve) 315 (M-H)
¹H NMR: δ (DMSO) 7.43-7.24 (5H, m), 4.40 (2H, s).

### d) (2R)-2-[[6-Chloro-5-nitro-2-[[phenylmethyl]thio]pyrimidin-4-yl]amino]-1-propanol

To a solution of the product of step (c) (6.4g) in tetrahydrofuran (60ml) and diisopropylethylamine (7.0ml) was added dropwise a solution of *R*-(-)-2-amino-1-propanol (1.5ml) in tetrahydrofuran (20ml) during 15 minutes. The mixture was stirred at room temperature for 18 hours. The mixture was concentrated under reduced pressure and the mixture poured into water (400ml) and extracted with ethyl acetate (three times), the combined organics dried (magmesium sulfate) and evaporated to a deep yellow gum (4.6g).
MS: APCI MW 355/357 (+H)
¹H NMR: δ (DMSO) 8.01 (1H, d), 7.40 (2H, d), 7.28 (2H, t), 7.25 (1H, t), 4.44 (1H, m), 4.36 (2H, d), 3.71 (1H, m), 3.60 (1H, m), 1.89 (1H, s), 1.26 (3H, d).

### e) (2R)-2-[[5-Nitro-2-[[phenylmethyl]thio]-6-thiocyanatopyrimidin-4-yl]amino]-1-propanol.

The product of step (d) (4.6g) and potassium thiocyanate (2.0g) were stirred in dry dimethylformamide (120ml) at room temperature for 18 hours. The mixture was poured into water (600ml) and extracted with ethyl acetate (three times) and the organics combined, washed with water (three times), dried (magnesium sulfate) and concentrated under reduced pressure to give a yellow solid (4.7g).
MS: APCI 376 (M-H), 378 (M+H)
¹H NMR: δ (DMSO) 9.06 (1H, d), 7.44 (2H, d), 7.29 (2H, m), 7.25 (1H, m), 5.10 (1H, s), 4.53 (2H, q), 3.53 (2H, m), 1.20 (3H, d).

### f) (2R)-2-[2-Amino-5-[[phenylmethyl]thio]thiazolo[5,4-d]pyrimidin-7-yl]amino-1-propanol.

The product of step (e) (4.7g), ammonium chloride (4.7g), and iron powder (1.4g) were heated to reflux in ethanol (200ml) and water (50ml) for 1 hour. The mixture was filtered hot and the filtrate concentrated under reduced pressure with the residue partitioned between ethyl acetate and water. The organic layer was collected and the aqueous layer extracted a further twice with ethyl acetate. The combined organics were shaken with water, dried (magnesium sulfate), and evaporated under reduced pressure to give the title compound as a fawn solid (4.3g).
MS: APCI MW 348 (M+H)
¹H NMR: δ (DMSO) 7.40 (4H, m), 7.28 (2H, t), 7.22 (1H, t), 6.50 (1H, d), 4.81 (1H, t), 4.32 (2H, q), 4.23 (1H, s), 3.42 (2H, m), 1.14 (3H, d).

### EXAMPLE 2

### (1R)-5-[[Phenylmethyl]thio]-7-[(2-hydroxy-1-methylethyl)amino]thiazolo[5,4-d]pyrimidin-2(1H)-one.

### a) (2R)-2-[2-Bromo-5-[[phenylmethyl]thio]thiazolo[5,4-d]pyrimidin-7-yl]amino-1-propanol.

To a suspension of the product of example 1 step (f) (0.54g) in bromoform (18ml) and acetonitrile (18ml) was added *tert*-butyl nitrite (0.23ml) for the mixture to be heated immediately at 60°C for 10 minutes. The red solution was quickly evaporated under reduced pressure to dryness in readiness to be purified by column chromatography (silica-4:1 dichloromethane/ethyl acetate) to give a solid (0.36g).
MS: APCI MW 409/411 (M-H), 411/413 (M+H).

### b) (2R)-2-[2-Methoxy-5-[[phenylmethyl]thio]thiazolo[5,4-d]pyrimidin-7-yl]amino-1-propanol.

The product of step (a) (0.36g) was suspended in methanol (25ml) and treated with potassium hydroxide powder (0.16g). The mixture was stirred overnight at room temperature and then acidified with concentrated hydrochloric acid to pH7 prior to being evaporated to dryness under reduced pressure.
MW: APCI MW 363 (M+H)

### c) (1R)-5-[[Phenylmethyl]thio]-7-[(2-hydroxy-1-methylethyl)amino]thiazolo[5,4-d]pyrimidin-2(1H)-one.

The product of step (b) was suspended in dioxan (40ml) followed by addition of concentrated hydrochloric acid (0.06ml) and water (0.06ml) to be stirred at 40°C for 2 days. After evaporation to dryness under reduced pressure, the residue was purified by column chromatography (silica - 3:2 dichloromethane/ethyl acetate).
MW: APCI MW 349 (M+H)
¹H NMR: δ (DMSO) 7.39 (2H, d), 7.30 (2H, t), 7.23 ( 1H, t), 6.81 (1H, d), 4.86 (1H, t), 4.32 (2H, t), 4.19 (1H, m), 3.44 (2H, t), 1.14 (3H, d).

### Example 3

### (2R)-2-[2-[(phenylmethyl)thio]-(1H-purin-6-yl)amino]-1-propanol

### a) 1,7-Dihydro-2-[(phenylmethyl)thio]-6H-purin-6-one

6-hydroxy-2-mercaptopurine (2g) was dissolved in a mixture of ethanol (10ml), 2N aqueous sodium hydroxide (11ml) and water (15ml). Under ice cooling and with stirring, benzyl bromide (2.1ml) was added to the solution and it was left stirring at room temperature for 30min. The solution was then cooled and neutralised with 2N HCl and diluted with 30ml isohexane. The resulting precipitate was filtered, washed with ethyl acetate and dried over P₂O₅ at 50°C for 2 hours to afford the subtitled product as a white solid (3.11 g).
m.p. 230-231°C
MS: APCI(+ve) 259 (M+1)
¹H NMR: δ (DMSO) 4.44 (s, 2H), 7.24-7.46 (m, 6H), 8.03 (s, 1H).

### b) 6-Chloro-2-[(phenylmethyl)thio]-1H-purine

The product of step (a) (4.4g) was placed in a three-necked flask with nitrogen flowing gently through one neck, a septum on the second and a condenser on the third. Phosphorus oxychloride (60ml) was added, then *N,N*-dimethylaniline (4ml) was added via syringe and the mixture was heated to 120°C in an oil bath for 2 hours. It was then allowed to cool overnight. The resulting mixture was poured on to ice with stirring and the ice was allowed to melt. The crude product was filtered off and washed with water and then purified using preparative HPLC to afford the subtitled product as a white solid (2.61 g).
m.p.175-176°C
MS: APCI(+ve) 277.5 (M+1)
¹H NMR: δ (DMSO) 4.45 (s, 2H), 7.23-7.49 (m, 5H), 8.54 (s, 1H) 13.78 (br s, 1H)

### c) (2R)-2-[2-[(Phenylmethyl)thio]-(1H-purin-6-yl)amino]-1-propanol

The product of step (b) (500mg) was mixed with D-alaninol (1ml) in N-methylpyrrolidinone (15ml), and the mixture was heated to 100°C for I 1 hours. The reaction mixture was cooled to room temperature, diluted with ethyl acetate (200ml), washed with brine (100ml), and the organic phase dried over MgSO₄, filtered and evaporated. The crude product was purified by preparative HPLC to give the title product as a white solid (224mg).
m.p. 208-209°C
MS: APCI(+ve) 316 (M+1)
¹H NMR: δ (DMSO) 1.16 (d, 3H), 3.32-3.54 (m, 2H), 4.37 (m, 3H), 4.77 (br s, 1H), 7.19-7.44 (m, 6H), 12.70 (br s, 1H), 7.98 (s, 1H).

### Example 4

### (2R)-2-[[6-[[(2,3-Difluorophenyl)methyl]thio]-1H-pyrazolo[3,4-d]pyrimidin-4-yl] amino]-1-propanol

### a) 6-[[(2,3-Difluorophenyl)methyl]thio]-1H-pyrazolo[3,4-d]pyrimidin-4-ol

6-mercapto-1*H*-pyrazolo[3,4-*d*]pyrimidin-4-ol (5g) was added to a suspension of 60% NaH (1.42g) in dry DMF (50ml) and left to stir for 1hour. 2,3-Difluorobenzyl bromide was then added slowly and the reaction mixture stirred for 1-2 hours. The resulting mixture was poured in to water whereupon the sub-titled product precipitated out. It was then filtered, washed with water and dried over P₂O₅ at 50°C for 10hrs (4.46g).
MS: APCI (+ve) 295 (M+1)
¹H NMR: δ (DMSO) 4.52 (s, 2H), 7.14-7.21 (m, 1H), 7.32-7.43 (m, 2H), 8.11 (br s, 1H), 12.34 (br s, 1H), 13.64 (br s, 1H).

### b) 4-Chloro-6-[[(2,3-difluorophenyl)methyl]thio]-1H-pyrazolo[3,4-d]pyrimidine

The product of step (a) (4.4g) was placed in a three-necked flask with nitrogen flowing gently through one neck, a septum on the second and a condenser on the third. Phosphorus oxychloride (60ml) was added, then *N, N*-dimethylaniline (6.0ml) added dropwise by syringe and the mixture was heated to 120°C in an oil bath for 2 hours. It was then allowed to cool overnight. The resulting mixture was poured on to ice with stirring and the ice was allowed to melt. The crude product was filtered off and washed with water and then purified using column chromatography eluting with DCM then 20% ethyl acetate/80% DCM. The appropriate fractions were evaporated to give the sub-titled product as a white solid (2.12g).
m.p. 191-192°C
MS: APCI (+ve) 314 (M+1)
¹H NMR: δ (DMSO) 4.55 (s, 2H), 7.13-7.21 (m, 1H), 7.31-7.46 (m, 2H), 8.34 (s, 1H), 14.31 (s, 1H).

### c) (2R)-2-[[6-[[(2,3-Difluorophenyl)methyl]thio]-1H-pyrazolo[3,4-d] pyrimidin-4-yl] amino]-1-propanol

The product of step (b) (250mg) was mixed with D-alaninol (1ml) in NMP (4ml), and the mixture was heated to 100°C for 11 hours. The reaction mixture was extracted with ethyl acetate (2x100ml) and brine (100ml), and the organic phase was dried over MgSO₄, filtered and evaporated. The crude product was purified by preparative HPLC to give the title product as a white solid (160mg).
m.p. 205-206°C
MS: APCI (+ve) 352 (M+1)
¹H NMR: δ (DMSO) 1.17 (d, 3H), 3.38-3.52 (m, 2H), 4.45 (s, 2H), 4.81 (t, 1H), 7.12-7.17 (m, 1H), 7.29-7.41 (m, 2H), 8.06 (s/d, 2H), 13.21 (s, 1H).

### Example 5

### 2-Methyl-2-[[5-[(phenylmethyl)thio]-3H-[1,2,3]-triazolo[4,5-d]pyrimidin-7-yl]amino]-1-propanol

### a) 2-(phenylmethyl)thio-4,6(1H,5H)-pyrimidinedione

To a solution of 4,6-dihydroxy-2-mercaptopyrimidine (100 g) in 5M NaOH (360 ml) was added first NMP (200 ml), then benzyl bromide (90 ml) dropwise over 2 hours. The reaction was stirred at room temperature for 24 hours, then acidified to pH 2 with conc HCl (100 ml) added dropwise over 2 hours at -5°C to give a pink precipitate. This was isolated by decanting off the solution followed by trituration with diethyl ether (1 litre) to give the subtitled product as a white powder after filtration and drying (210 g).
m.p. 220-250°C (dec)
MS: APCI (-ve) 233 (M-H)
¹H NMR: δ (DMSO) 11.76 (br s, 1H), 7.45 (d, 2H), 7.26 (m, 3H), 5.18 (s, 1H), 4.38 (s, 2H).

### b) 4,6-Dihydroxy-5-nitro-2-[(phenylmethyl)thio]pyrimidine

The product of step (a) (2 g) was added to a mixture of glacial acetic acid (50 ml) and concentrated nitric acid (20 ml) and the reaction mixture heated to 50°C. A further 28 g of the product of step (a) was added in portions over 2 hours whilst maintaining the reaction temperature between 50 and 60°C. After stirring the reaction mixture for a further 1 hour at 50°C it was poured onto crushed ice and the subtitled product isolated by filtration as a yellow solid (12.3 g).
¹H NMR: δ (DMSO) 7.48-7.19 (m, 5H), 4.47 (s, 2H).

### c) 4,6-dichloro-5-nitro-2-[(phenylmethyl)thio]pyrimidine

A suspension of the product of step (b) ( 59.2 g) in a mixture of phosphorous oxychloride (100 ml) and toluene (400 ml) was heated to 80°C. A solution of 1-methylimidazole (16.9 ml) in toluene (200 ml) was added dropwise over 1 hour and the reaction mixture then heated at 100°C for 24 hours. After cooling to room temperature, the solvent was removed by evaporation and water (2 litres) cautiously added to the residue. The mixture was extracted with dichloromethane (4x500 ml) and the combined organic extracts dried over MgSO₄, filtered and evaporated to give a brown tar. This was purified by column chromatography, eluting with 10% dichloromethane in isohexane, to afford the subtitled product as a yellow solid (29.7 g).
MS: APCI (-ve) 315 (M-H)
¹H NMR: δ (DMSO) 7.43-7.24 (m, 5H), 4.40 (s, 2H).

### d) 4-Amino-6-chloro-5-nitro-2-[(phenylmethyl)thio]pyrimidine

To a solution of the product of step (c) (1 g) in diethyl ether (20 ml) was added first *N*,*N*'-diisopropylethylamine (1.1 ml) followed by a solution of methanolic 7N ammonia (1.2 ml) in diethyl ether (10 ml) dropwise over 30 minutes. The reaction mixture was stirred at room temperature for 24 hours, then acidified to pH 2 by the addition of conc hydrochloric acid (5 ml). The organic phase was separated, dried over MgSO₄, filtered and evaporated to afford the subtitled product as a yellow solid (0.58 g).
¹H NMR: δ (DMSO) 7.40-7.25 (m, 5H), 4.36 (s, 2H).

### e) 2-[[(6-Amino-5-nitro-2-[(phenylmethyl)thio]-4-pyrimidinyl]amino]-2-methyl-1-propanol

2-Amino-2-methyl-1-propanol (0.09 ml) was added to a solution of the product of step (d) (100 mg) in DMF (7 ml) and the reaction mixture stirred at room temperature for 24 hours. The solvent was removed by evaporation and the residue purified by column chromatography, eluting with 30% ethyl acetate in isohexane, to afford the subtitled product as a yellow solid (106 mg).
MS: APCI (+ve) 349 (M⁺)
¹H NMR: δ (DMSO) 9.67 (s, I H), 8.69 (s, 1H), 8.60 (s, 1H), 7.43-7.24 (m, 5H), 5.21 (t, 1H), 4.37 (s, 2H), 3.45 (d, 2H), 1.37 (s, 6H).

### f) 2-[[[5,6-diamino-2-(phenylmethyl)thio]-4-pyrimidinyl]amino]-2-methyl-1-propanol

Sodium hydrosulphite (9.3 g) was added in portions to a solution of the product of step (e) in 1,4-dioxane (60 ml) at 55°C. The reaction mixture was stirred vigorously at this temperature whilst water (50 ml) was added dropwise over 30 minutes. The 1,4-dioxane was removed by evaporation giving a white precipitate, which was isolated by filtration. This was purified by column chromatography, eluting with 25% dichloromethane in ethyl acetate, to afford the subtitled product as a white solid (0.67 g).
¹H NMR: δ (DMSO) 7.40-7.20 (m, 5H), 5.77 (s, 2H), 5.35 (s, 1H), 5.06 (t, 1H), 4.25 (s, 2H), 3.48 (s, 2H), 1.29 (s, 6H).

### g) 2-Methyl-2-[[5-[(phenylmethyl)thio]-3H-[1,2,3]-triazolo[4,5-d]pyrimidin-7-yl]amino]-1-propanol

Isoamyl nitrite (0.41 ml) was added to a suspension of the product of step (f) in in acetonitrile (50 ml) and the reaction mixture heated at reflux for 30 minutes. The solvent was evaporated and the residue purified by column chromatography, eluting with 3% methanol in dichloromethane, to afford the title compound as a white solid which was further purified by recrystallization from ethyl acetate (43 mg).
m.p. 191°C
MS: APCI (+vc) 330 (M⁺)
¹H NMR: δ (DMSO) 7.43-7.21 (m, 5H), 4.87 (br s, 1H), 4.42 (s, 2H), 3.60 (s, 2H), 1.28 (s, 6H).

### Pharmacological Data

### Ligand Binding Assay

[¹²⁵I]IL-8 (human, recombinant) was purchased from Amcrsham, U.K. with a specific activity of 2,000Ci/mmol. All other chemicals were of analytical grade. High levels of hrCXCR2 were expressed in HEK 293 cells (human embryo kidney 293 cells ECACC No. 85120602) (Lee *et al*. (1992) *J. Biol. Chem*. **267** pp 16283-16291). hrCXCR2 cDNA was amplified and cloned from human neutrophil mRNA. The DNA was cloned into PCRScript (Stratagene) and clones were identified using DNA. The coding sequence was sub-cloned into the eukaryotic expression vector RcCMV (Invitrogen). Plasmid DNA was prepared using Quiagen Megaprep 2500 and transfected into HEK 293 cells using Lipofectamine reagent (Gibco BRL). Cells of the highest expressing clone were harvested in phosphate-buffered saline containing 0.2%(w/v) ethylenediaminetetraacetic acid (EDTA) and centrifuged (200g, 5min.). The cell pellet was resuspended in ice cold homogenisation buffer [10mM HEPES (pH 7.4), 1mM dithiothreitol, 1mM EDTA and a panel of protease inhibitors (1mM phenyl methyl sulphonyl fluoride, 2µg/ml soybean trypsin inhibitor, 3mM benzamidine, 0.5µg/ml leupeptin and 100µg/ml bacitracin)] and the cells left to swell for 10 minutes. The cell preparation was disrupted using a hand held glass mortar/PTFE pestle homogeniser and cell membranes harvested by centrifugation (45 minutes, 100,000g, 4°C). The membrane preparation was stored at -70°C in homogenisation buffer supplemented with Tyrode's salt solution (137mM NaCl, 2.7mM KCl, 0.4mM NaH₂PO₄), 0.1%(w/v) gelatin and 10%(v/v) glycerol.

All assays were performed in a 96-well MultiScreen 0.45µm filtration plates (Millipore, U.K.). Each assay contained ∼50pM [¹²⁵I]IL-8 and membranes (equivalent to ~200,000 cells) in assay buffer [Tyrode's salt solution supplemented with 10mM HEPES (pH 7.4), 1.8mM CaCl₂, 1mM MgCl₂, 0.125mg/ml bacitracin and 0.1%(w/v) gelatin]. In addition, a compound of formula (I) according to the Examples was pre-dissolved in DMSO and added to reach a final concentration of 1%(v/v) DMSO. The assay was initiated with the addition of membranes and after 1.5 hours at room temperature the membranes were harvested by filtration using a Millipore MultiScreen vacuum manifold and washed twice with assay buffer (without bacitracin). The backing plate was removed from the MultiScreen plate assembly, the filters dried at room temperature, punched out and then counted on a Cobra γ-counter.

The compounds of formula (I) according to the Examples were found to have IC₅₀ values of less than (<) 10µM.

### Intracellular Calcium Mobilisation Assay

Human neutrophils were prepared from EDTA-treated peripheral blood, as previously described (Baly *et al.* (1997) Methods in Enzymology 287 pp70-72), in storage buffer [Tyrode's salt solution (137mM NaCl, 2.7mM KCl, 0.4mM NaH₂PO₄) supplemented with 5.7mM glucose and 10mM HEPES (pH 7.4)].

The chemokine GROα (human, recombinant) was purchased from R&D Systems (Abingdon, U.K.). All other chemicals were of analytical grade. Changes in intracellular free calcium were measured fluorometrically by loading neutrophils with the calcium sensitive fluorescent dye, fluo-3, as described previously (Merritt *et al*. (1990) Biochem. J. 269, pp513-519). Cells were loaded for 1 hour at 37°C in loading buffer (storage buffer with 0.1%(w/v) gelatin) containing 5µM fluo-3 AM ester, washed with loading buffer and then resuspended in Tyrode's salt solution supplemented with 5.7mM glucose, 0.1%(w/v) bovine serum albumin (BSA), 1.8mM CaCl₂ and 1mM MgCl₂. The cells were pipetted into black walled, clear bottom, 96 well micro plates (Costar, Boston, U.S.A.) and centrifuged (200g, 5 minutes, room temperature).

A compound of formula (I) according to the Examples was pre-dissolved in DMSO and added to a final concentration of 0.1 %(v/v) DMSO. Assays were initiated by the addition of an A₅₀ concentration of GROα and the transient increase in fluo-3 fluorescence (λ_{Ex} =490nm and λ_{Em} = 520nm) monitored using a FLIPR (Fluorometric Imaging Plate Reader, Molecular Devices, Sunnyvale, U.S.A.).

The compounds of formula (I) according to the Examples were tested and found to be antagonists of the CXCR2 receptor in human neutrophils.

## Claims

1. A compound of formula (I) or pharmaceutically acceptable salts or solvates thereof: 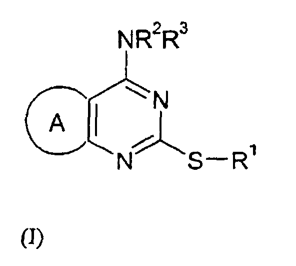 in which:
A is a group of formula (a), (b) or (c): 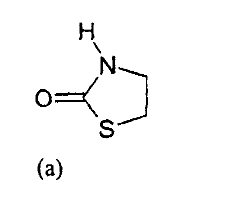 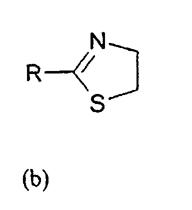 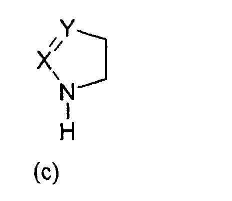 in which:
R¹ represents an optionally branched C₁-C₄ alkyl group which terminates in an aryl or heteroaryl group each of which can be optionally substituted by one or more substituents independently selected from halogen atoms, cyano, nitro, -OR⁴, -NR⁵R⁶, -CONR⁵R⁶, -COOR⁷, -NR⁸COR⁹, -SR⁹, -SO₂R⁹, -SO₂NR⁵R⁶, -NR⁸SO₂R⁹, C₁-C₆ alkyl or trifluoromethyl groups and which may optionally contain one or more atoms independently selected from, O, NR⁵, or S.
R² represents an optionally branched C₁-C₈ alkyl group terminating in an OH substituent which may be optionally substituted by one or more substituent groups independently selected from -OR⁴, -NR⁵R⁶ -CONR⁵R⁶, -NR⁸COR⁹, -SR⁹, -SO₂R⁹, -SO₂NR⁵R⁶, -NR⁸SO₂R⁹,
R³ represents hydrogen or C₁-C₆ alkyl optionally substituted by one or more substituent groups independently selected from halogen atoms, phenyl, -OR¹⁰ and -NR¹¹R¹²,
R⁴ represents hydrogen, C₁-C₆ alkyl or a phenyl group the latter two of which may be optionally substituted by one or more substituent groups independently selected from halogen atoms, phenyl, -OR¹⁰ and -NR⁵R⁶.
R⁵ and R⁶ independently represent a hydrogen atom or a C₁-C₆ alkyl or phenyl group the latter two of which may be optionally substituted by one or more substituent groups independently selected from halogen atoms, phenyl, -OR¹³ and -NR¹⁴R¹⁵, -CONR¹⁴R¹⁵, -NR¹⁴COR¹⁵, -SO₂NR¹⁴R¹⁵, NR¹⁴SO₂R¹⁵
or
R⁵ and R⁶ together with the nitrogen atom to which they are attached form a 4- to 7-membered saturated heterocyclic ring system optionally comprising a further heteroatom selected from oxygen and nitrogen atoms, which ring system may be optionally substituted by one or more substituent groups independently selected from phenyl, -OR¹³, -COOR¹³, - NR¹⁴R¹⁵, -CONR¹⁴R¹⁵, -NR¹⁴COR¹⁵, -SO₂NR¹⁴R¹⁵, -NR¹⁴SO₂R¹⁵ or C₁-C₆ alkyl, itself optionally substituted by one or more substituents independently selected from halogen atoms and -NR¹⁴R¹⁵ and -OR¹⁶ groups,
X represents a nitrogen atom or CH ;
Y represents a nitrogen atom or CH;
R represents a hydrogen atom, or a group -NR¹⁷R¹⁸;
R¹⁷ and R¹⁸ each independently represent a hydrogen atom, or a 4-piperidinyl, C₃-C₆ cycloalkyl or C₁-C₈ alkyl group, which latter two groups may be optionally substituted by one or more substituent groups independently selected from halogen atoms and -NR⁵R⁶,-CONR⁵R⁶, -OR⁹, -COOR⁷, -NR⁸COR⁹, -SR¹¹, -SO₂R¹¹, -SO₂NR⁵R⁶, -NR⁸SO₂R⁹, morpholinyl, C₁-C₄ alkyl, C₃-C₆ cycloalkyl, tetrahydrofuranyl, aryl and heteroaryl groups, each of the latter two groups may be optionally substituted by one or more substituents independently selected from halogen atoms, cyano, nitro, -NR⁵R⁶, -CONR⁵R⁶, -OR⁹, -NR⁸COR⁹, -SO₂NR⁵R⁶, -NR⁸SO₂R⁹, C₁-C₆ alkyl and trifluoromethyl groups where R⁵, R⁶, R⁸ and R⁹ are as defined,
or R¹⁷ and R¹⁸ together with the nitrogen atom to which they are attached form a 4- to 7-membered saturated heterocyclic ring system, which ring system may be optionally substituted by one or more substituent groups independently selected from 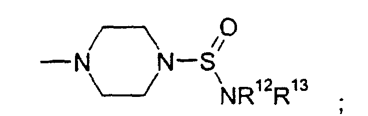
-NR⁵R⁶, -CONR⁵R⁶, -OR⁹, -COOR⁷, -NR⁸COR⁹, and C₁-C₆ alkyl optionally substituted by one or more substituents independently selected from halogen atoms and -NR¹²R¹³ and -OR⁸ groups,
R⁹ represents a hydrogen atom, a C₁-C₆ alkyl group or a phenyl group, each of which may be optionally substituted by one or more substituent groups independently selected from halogen atoms, phenyl, -OR¹⁶ and -NR¹⁴R¹⁵, and
each of R⁷, R⁸, R¹⁰, R¹¹, R¹², R¹³, R¹⁴ R¹⁵, and R¹⁶ independently represents a hydrogen atom or a C₁-C₆, alkyl, or a phenyl group,
• provided that when Y is CH, then X is N.

2. A compound according to claim 1 in which A represents a group of formula (a) or (c).

3. A compound according to claim 1 or 2 wherein R¹ represents an optionally substituted benzyl group.

4. A compound according to any one of claims 1 to 3 wherein R² is CH(CH₃)CH₂OH, CH(Et)CH₂OH or C(CH₃)₂CH₂OH.

5. A compound according to any one of claims 1 to 4 wherein R³ is hydrogen.

6. A compound according to claim 1 selected from:
(2*R*)-2-[[2-Amino-5-[(phenylmethyl)thio]thiazolo[5,4-*d*]pyrimidin-7-yl]amino]-1-propanol.
(1*R*)-5-[[Phenylmethyl]thio]-7-[(2-hydroxy-1-methylethyl)amino]thiazolo[5,4-*d*]pyrimidin-2(1*H*)-one.
(2*R*)-2-[2-[(phenylmethyl)thio]-(1*H*-purin-6-yl)amino]-1-propanol
(2*R*)-2-[[6-[[(2,3-Difluorophenyl)methyl]thio]-1*H*-pyrazolo[3,4-*d*]pyrimidin-4-yl] amino]-1-propanol and
2-Methyl-2-[[5-[(phenylmethyl)thio]-3*H*-[1,2,3]-triazolo[4,5-*d*]pyrimidin-7-yl]amino]-1-propanol
and their pharmaceutically acceptable salts and solvates.

7. A process for the preparation of a compound of formula (I) as defined in claim 1 which comprises:
(I) for compounds of formula (I) in which A is a group (a), treatment of a compound of formula (II): 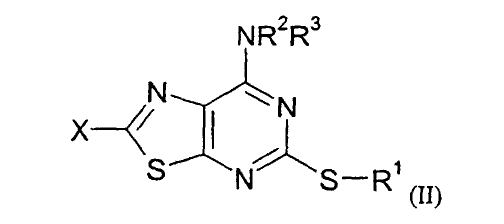 where R¹, R² and R³ are as defined in formula (I) or are protected derivatives thereof and X is a leaving group with aqueous acid, or
(2) for compounds of formula (I) in which A is a group of formula (c), treatment of a compound of formula (IIA): 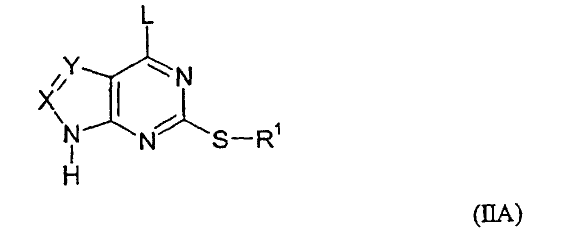 where X, Y and R¹ are as defined in formula (I) or are protected derivatives thereof and L is a leaving group with an amine HNR²R³, or
(3) for compounds of formula (I) in which A is a group of formula (c) and X and Y are both N, reacting a compound of general formula (IIB): 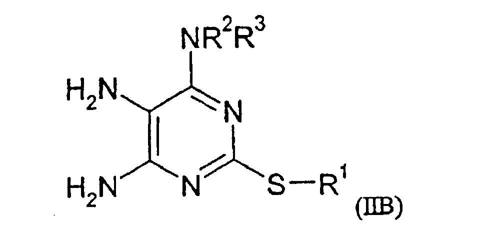 wherein R¹, R² and R³ are as defined in formula (I) or are protected derivatives thereof with a nitrosating agent, or
(4) for compounds of formula (I) A is a group (b) and where R¹, R² and R³ are as defined in formula (I) or are protected derivatives thereof and R is NH₂, treating a compound of formula (III) with a reducing agent: 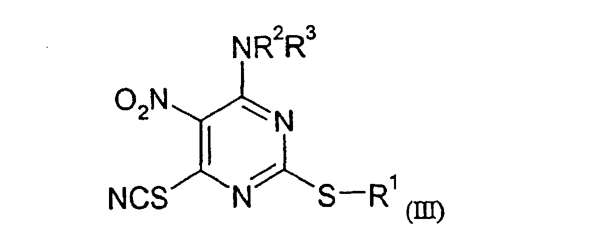 and optionally thereafter any of processes 1 to 4:
• removing any protecting groups present
• forming a pharmaceutically acceptable salt or solvate.

8. An intermediate compound of formula (IIB) as defined in claim 7.

9. A pharmaceutical composition comprising a compound of formula (I), or a pharmaceutically acceptable salt or solvate thereof, as claimed in any one of claims 1 to 6 in association with a pharmaceutically acceptable adjuvant, diluent or carrier.

10. A process for the preparation of a pharmaceutical composition as claimed in claim 9 which comprises mixing a compound of formula (I), or a pharmaceutically acceptable salt or solvate thereof, as claimed in any one of claims 1 to 6 with a pharmaceutically acceptable adjuvant, diluent or carrier.

11. A compound of formula (I), or a pharmaceutically acceptable salt or solvate thereof, as claimed in any one of claims 1 to 6 for use in therapy.

12. A compound according to claim 11 for use in a method of treating a chemokine mediated disease wherein the chemokine binds to one or more chemokine receptors, which comprises administering to a patient a therapeutically effective amount of a compound of formula (I), or a pharmaceutically acceptable salt or solvate thereof, as claimed in any one of claims 1 to 6.

13. A compound according to claim 12 in which the chemokine receptor belongs to the CXC chemokine receptor subfamily.

14. A compound according to claim 12 or 13 in which the chemokine receptor is the CXCR2 receptor.

15. A compound according to claim 11 for use in a method of treating an inflammatory disease in a patient suffering from, or at risk of, said disease, which comprises administering to the patient a therapeutically effective amount of a compound of formula (I), or a pharmaceutically acceptable salt or solvate thereof, as claimed in any one of claims 1 to 6.

16. A compound according to claim 15, wherein the disease is psoriasis, a disease in which angiogenesis is associated with raised CXCR2 chemokine levels, or COPD.

17. A compound according to claim 16, wherein the disease is psoriasis.

18. A compound, salt or solvate according to claim 11 wherein the disease is cancer.

19. A compound, salt or solvate according to claim 11 wherein the disease is of the gastrointestinal tract.

20. A compound, salt or solvate according to claim 11 wherein the disease is COPD.

## Patentansprüche

1. Verbindung der Formel (I) oder pharmazeutisch unbedenkliche Salze oder Solvate davon: 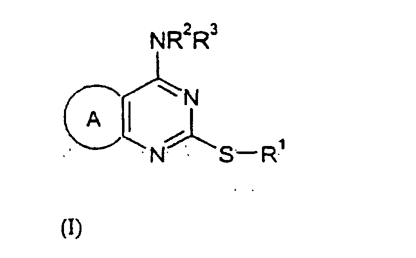 worin:
A für eine Gruppe der Formel (a), (b) oder (c): 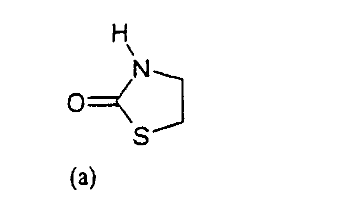 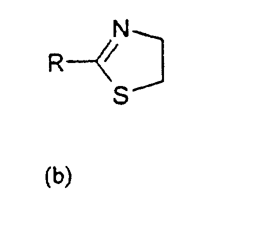 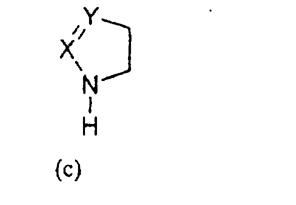 steht, worin:
R¹ für eine gegebenenfalls verzweigte C₁-C₄-Alkylgruppe mit einer endständigen Aryl- oder Heteroarylgruppe, die gegebenenfalls durch einen oder mehrere, unabhängig voneinander unter Halogenatomen, Cyano-, Nitro-, -OR⁴-, -NR⁵R⁶-, -CONR⁵R⁶-, -COOR⁷-, -NR⁸COR⁹-, -SR⁹-, -SO₂R⁹-, -SO₂NR⁵R⁶-, -NR⁸SO₂R⁹-, C₁-C₆-Alkyl- oder Trifluormethylgruppen ausgewählte Substituenten substituiert sein kann und gegebenenfalls ein oder mehrere, unabhängig voneinander unter O, NR⁵ oder S ausgewählte Atome enthalten kann, steht,
R² für eine gegebenenfalls verzweigte C₁-C₈-Alkylgruppe mit einem endständigen OH-Substituenten, die gegebenenfalls durch einen oder mehrere, unabhängig voneinander unter -OR⁴, -NR⁵R⁶, -CONR⁵R⁶, -NR⁸COR⁹, -SR⁹, -SO₂R⁹, -SO₂NR⁵R⁶, -NR⁸SO₂R⁹ ausgewählte Substituentengruppen substituiert sein kann, steht,
R³ für Wasserstoff oder gegebenenfalls durch einen oder mehrere, unabhängig voneinander unter Halogenatomen, Phenyl, -OR¹⁰ und -NR¹¹R¹² ausgewählte Substituentengruppen substituiertes C₁-C₆-Alkyl steht,
R⁴ für Wasserstoff, C₁-C₆-Alkyl oder eine Phenylgruppe steht, wobei die beiden letztgenannten Gruppen gegebenenfalls durch einen oder mehrere, unabhängig voneinander unter Halogenatomen, Phenyl, -OR¹⁰ und -NR⁵R⁶ ausgewählte Substituentengruppen substituiert sein können,
R⁵ und R⁶ unabhängig voneinander für ein Wasserstoffatom oder eine C₁-C₆-Alkyl- oder Phenylgruppe stehen, wobei die beiden letztgenannten Gruppen gegebenenfalls durch eine oder mehrere, unabhängig voneinander unter Halogenatomen, Phenyl, -OR¹³ und -NR¹⁴R¹⁵, -CONR¹⁴R¹⁵, -NR¹⁴COR¹⁵, -SO₂NR¹⁴R¹⁵ oder NR¹⁴SO₂R¹⁵ ausgewählte Substituentengruppen substituiert sein können,
oder
R⁵ und R⁶ gemeinsam mit dem Stickstoffatom, an das sie gebunden sind, ein 4- bis 7-gliedriges gesättigtes heterocyclisches Ringsystem bilden, welches gegebenenfalls ein weiteres, unter Sauerstoff- und Stickstoffatomen ausgewähltes Heteroatom enthalten kann und gegebenenfalls durch eine oder mehrere, unter Phenyl, -OR¹³, -COOR¹³; -NR¹⁴R¹⁵, -CONR¹⁴R¹⁵, -NR¹⁴COR¹⁵, -SO₂NR¹⁴R¹⁵, -NR¹⁴SO₂R¹⁵ oder C₁-C₆-Alkyl, welches selbst gegebenenfalls durch einen oder mehrere, unabhängig voneinander unter Halogenatomen und -NR¹⁴R¹⁵- und -OR¹⁶-Gruppen ausgewählte Substituenten substituiert sein kann, Substituentengruppen substituiert sein kann,
X für ein Stickstoffatom oder CH steht,
Y für ein Stickstoffatom oder CH steht,
R für ein Wasserstoffatom oder eine Gruppe -NR¹⁷R¹⁸ steht,
R¹⁷ und R¹⁸ jeweils unabhängig voneinander für ein Wasserstoffatom oder eine 4-Piperidinyl-, C₃-C₆-Cycloalkyl- oder C₁-C₈-Alkylgruppe stehen, wobei die beiden letztgenannten Gruppen gegebenenfalls durch eine oder mehrere, unabhängig voneinander unter Halogenatomen und -NR⁵R⁶-, -CONR⁵R⁶-, -OR⁹-, -COOR⁷-, -NR⁸COR⁹-, -SR¹¹-, -SO₂R¹¹-, -SO₂NR⁵R⁶-, -NR⁸SO₂R⁹-, Morpholinyl-, C₁-C₄-Alkyl-, C₃-C₆-Cycloalkyl-, Tetrahydrofuranyl-, Aryl- und Heteroarylgruppen ausgewählte Substituentengruppen substituiert sein können, wobei jede der beiden letztgenannten Gruppen gegebenenfalls durch einen oder mehrere, unabhängig voneinander unter Halogenatomen, Cyano-, Nitro-, -NR⁵R⁶-, -CONR⁵R⁶-, -OR⁹-, -NR⁸COR⁹-, -SO₂NR⁵R⁶-, -NR⁸SO₂R⁹-, C₁-C₆-Alkyl- und Trifluormethylgruppen, wobei R⁵, R⁶, R⁸ und R⁹ die angegebene Bedeutung besitzen, ausgewählte Substituenten substituiert sein kann,
oder
R¹⁷ und R¹⁸ gemeinsam mit dem Stickstoffatom, an das sie gebunden sind, ein 4- bis 7-gliedriges gesättigtes heterocyclisches Ringsystem bilden, welches gegebenenfalls durch eine oder mehrere, unabhängig voneinander unter 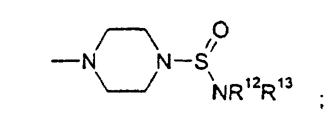 -NR⁵R⁶, -CONR⁵R⁶, -OR⁹, -COOR⁷, -NR⁸COR⁹ und gegebenenfalls durch ein oder mehrere, unabhängig voneinander unter Halogenatomen und -NR¹²R¹³- und -OR⁸-Gruppen ausgewählte Substituenten substituiertem C₁-C₆-Alkyl ausgewählte Substituentengruppen substituiert sein kann,
R⁹ für ein Wasserstoffatom, eine C₁-C₆-Alkylgruppe oder eine Phenylgruppe steht, die jeweils gegebenenfalls durch eine oder mehrere, unabhängig voneinander unter Halogenatomen, Phenyl, -OR¹⁶ und -NR¹⁴R¹⁵ ausgewählte Substituentengruppen substituiert sein können und
R⁷, R⁸, R¹⁰, R¹¹, R¹², R¹³, R¹⁴, R¹⁵ und R¹⁶ jeweils unabhängig voneinander für ein Wasserstoffatom oder eine C₁-C₆-Alkyl- oder eine Phenylgruppe stehen,
• mit der Maßgabe, daß X für N steht, wenn Y für CH steht.

2. Verbindung nach Anspruch 1, worin A für eine Gruppe der Formel (a) oder (c) steht.

3. Verbindung nach Anspruch 1 oder 2, worin R¹ für eine gegebenenfalls substituierte Benzylgruppe steht.

4. Verbindung nach einem der Ansprüche 1 bis 3, worin R² für CH(CH₃)CH₂OH, CH(Et)CH₂OH oder C(CH₃)₂CH₂OH steht.

5. Verbindung nach einem der Ansprüche 1 bis 4, worin R³ für Wasserstoff steht.

6. Verbindung nach Anspruch 1, ausgewählt unter:
(2*R*)-2-[[2-Amino-5-[(phenylmethyl)thio]thiazolo-[5,4-*d*]pyrimidin-7-yl]amino]-1-propanol,
(1*R*)-5-[[Phenylmethyl]thio]-7-[(2-hydroxy-1-methylethyl)amino]thiazolo[5,4-*d*]pyrimidin-2(1*H*)-on,
(2*R*)-2-[2-[(Phenylmethyl)thio]-(1*H*-purin-6-yl) amino]-1-propanol,
(2*R*)-2-[[6-[[(2,3-Difluorphenyl)methyl]thio]-1*H*pyrazolo[3,4-*d*]pyrimidin-4-yl]amino]-1-propanol und 2-Methyl-2-[[5-[(phenylmethyl)thio]-3*H*-[1,2,3] triazolo[4,5-*d*]pyrimidin-7-yl]amino]-1-propanol
und pharmazeutisch unbedenklichen Salzen und Solvaten davon.

7. Verfahren zur Herstellung einer Verbindung der Formel (I) nach Anspruch 1, bei dem man:
(1) zur Herstellung von Verbindungen der Formel (I), in denen A für eine Gruppe (a) steht, eine Verbindung der Formel (II): 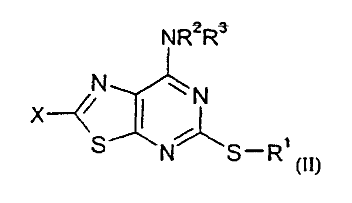 worin R¹, R² und R³ die unter Formel (I) angegebene Bedeutung besitzen oder für geschützte Derivate davon stehen und X für eine Abgangsgruppe steht, mit wäßriger Säure behandelt, oder
(2) zur Herstellung von Verbindungen der Formel (I), in denen A für eine Gruppe (c) steht, eine Verbindung der Formel (IIA): 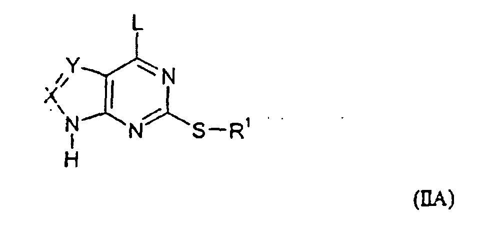 worin X, Y und R¹ die unter Formel (I) angegebene Bedeutung besitzen oder für geschützte Derivate davon stehen und L für eine Abgangsgruppe steht, mit einem Amin HNR²R³ behandelt, oder
(3) zur Herstellung von Verbindungen der Formel (I), in denen A für eine Gruppe (c) steht und X und Y beide für N stehen, eine Verbindung der allgemeinen Formel (IIB): 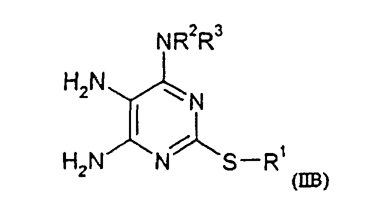 worin R¹, R² und R³ die unter Formel (I) angegebene Bedeutung besitzen oder für geschützte Derivate davon stehen, mit einem Nitrosierungsmittel behandelt, oder
(4) zur Herstellung von Verbindungen der Formel (I), in denen A für eine Gruppe (b) steht und R¹, R² und R³ die unter Formel (I) angegebene Bedeutung besitzen oder für geschützte Derivate davon stehen und R für NH₂ steht, eine Verbindung der Formel (III) mit einem Reduktionsmittel behandelt: 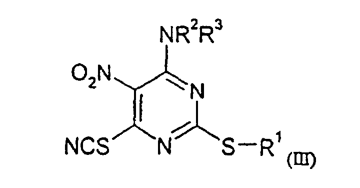 und gegebenenfalls nach einem der Verfahren 1 bis 4:
• jegliche vorhandenen Schutzgruppen abspaltet
• ein pharmazeutisch unbedenkliches Salz oder Solvat herstellt.

8. Zwischenprodukt der Formel (IIB) gemäß Anspruch 7.

9. Pharmazeutische Zusammensetzung, enthaltend eine Verbindung der Formel (I) oder ein pharmazeutisch unbedenkliches Salz oder Solvat davon nach einem der Ansprüche 1 bis 6 zusammen mit einem pharmazeutisch unbedenklichen Hilfsstoff, Verdünnungsmittel oder Träger.

10. Verfahren zur Herstellung einer pharmazeutischen Zusammensetzung nach Anspruch 9, bei dem man eine Verbindung der Formel (I) oder ein pharmazeutisch unbedenkliches Salz oder Solvat davon nach einem der Ansprüche 1 bis 6 mit einem pharmazeutisch unbedenklichen Hilfsstoff, Verdünnungsmittel oder Träger vermischt.

11. Verbindung der Formel (I) oder ein pharmazeutisch unbedenkliches Salz oder Solvat davon nach einem der Ansprüche 1 bis 6 zur Verwendung bei der Therapie.

12. Verbindung nach Anspruch 11 zur Verwendung bei einem Verfahren zur Behandlung einer Chemokinvermittelten Erkrankung, bei der das Chemokin an einen oder mehrere Chemokinrezeptoren bindet, bei dem man einem Patienten eine therapeutisch wirksame Menge einer Verbindung der Formel (I) oder eines pharmazeutisch unbedenklichen Salzes oder Solvats davon nach einem der Ansprüche 1 bis 6 verabreicht.

13. Verbindung nach Anspruch 12, wobei der Chemokinrezeptor zur Unterfamilie der CXC-Chemokinrezeptoren gehört.

14. Verbindung nach Anspruch .12 oder 13, wobei es sich bei dem Chemokinrezeptor um den CXCR2-Rezeptor handelt.

15. Verbindung nach Anspruch 11 zur Verwendung bei einem Verfahren zur Behandlung einer entzündlichen Erkankung bei einem Patienten, der an dieser Erkrankung leidet oder bei dem das Risiko dieser Erkrankung besteht, bei dem man einem Patienten eine therapeutisch wirksame Menge einer Verbindung der Formel (I) oder eines pharmazeutisch unbedenklichen Salzes oder Solvats davon nach einem der Ansprüche 1 bis 6 verabreicht.

16. Verbindung nach Anspruch 15, wobei es sich bei der Erkrankung um Psoriasis, eine Erkrankung, bei der die Angiogenese mit erhöhten CXCR2-Chemokin-Spiegeln assoziiert ist, oder COPD handelt.

17. Verbindung nach Anspruch 16, wobei es sich bei der Erkrankung um Psoriasis handelt.

18. Verbindung, Salz oder Solvat nach Anspruch 11, wobei es sich bei der Erkrankung um Krebs handelt.

19. Verbindung, Salz oder Solvat nach Anspruch 11, wobei die Erkrankung den Magen-Darm-Trakt betrifft.

20. Verbindung, Salz oder Solvat nach Anspruch 11, wobei es sich bei der Erkrankung um COPD handelt.

## Revendications

1. Composé de formule (I) ou les sels ou solvates pharmaceutiquement acceptables de celui-ci : 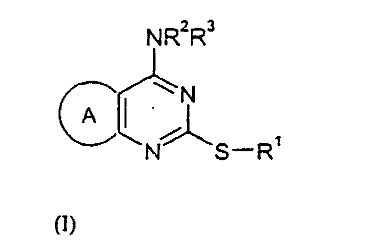 dans laquelle :
A est un groupement de formules (a), (b) ou (c) : 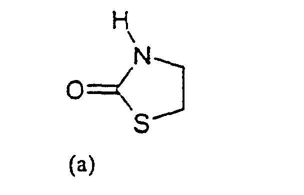 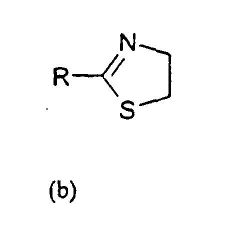 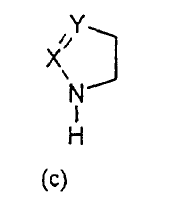 dans lesquelles :
R¹ représente un groupement alkyle en C₁-C₄ éventuellement ramifié se terminant par un groupement aryle ou hétéroaryle dont chacun peut être éventuellement substitué par un ou plusieurs substituants choisis indépendamment parmi les atomes d'halogène, ou les groupements cyano, nitro, -OR⁴, -NR⁵R⁶, -CONR⁵R⁶, -COOR⁷, -NR⁸COR⁹-, -SR⁹, -SO₂R⁹, -SO₂NR⁵R⁶, -NR⁸SO₂R⁹, alkyle en C₁-C₆ ou trifluorométhyle et pouvant éventuellement contenir un ou plusieurs atomes choisis indépendamment parmi O, NR⁵ ou S ;
R² représente un groupement alkyle en C₁-C₈ éventuellement ramifié se terminant par un substituant OH pouvant être éventuellement substitué par un ou plusieurs groupements substituants choisis indépendamment parmi -OR⁴, - NR⁵R⁶ -CONR⁵R⁶, -NR⁸COR⁹, -SR⁹, -SO₂R⁹, -SO₂NR⁵R⁶, - NR⁸SO₂R⁹ ;
R³ représente hydrogène ou alkyle en C₁-C₆ éventuellement substitué par un ou plusieurs groupements substituants choisis indépendamment parmi les atomes d'halogène, phényle, -OR¹⁰ et -NR¹¹R¹² ;
R⁴ représente hydrogène, ou groupement alkyle en C₁-C₆ ou phényle, dont les deux derniers groupements peuvent être éventuellement substitués par un ou plusieurs groupements substituants choisis indépendamment parmi les atomes d'halogène, phényle, -OR¹⁰ et -NR⁵R⁶ ;
R⁵ et R⁶ représentent indépendamment un atome d'hydrogène ou un groupement alkyle en C₁-C₆ ou phényle, dont les deux derniers peuvent être éventuellement substitués par un ou plusieurs groupements substituants choisis indépendamment parmi les atomes d'halogène, phényle, -OR¹³ et -NR¹⁴R¹⁵, -CONR¹⁴R¹⁵, -NR¹⁴COR¹⁵, -SO₂NR¹⁴R¹⁵, NR¹⁴SO₂R¹⁵
ou
R⁵ et R⁶, pris ensemble avec l'atome d'azote auquel ils sont liés, forment un système de noyaux hétérocycliques saturé à 4 à 7 chaînons comprenant éventuellement un autre hétéroatome choisi parmi les atomes d'oxygène et d'azote, lequel système de noyaux peut éventuellement être substitué par un ou plusieurs groupements substituants choisis indépendamment parmi phényle, -OR¹³, -COOR¹³, -NR¹⁴R¹⁵, -CONR¹⁴R¹⁵, -NR¹⁴COR¹⁵, -SO₂NR¹⁴R¹⁵, -NR¹⁴SO₂R¹⁵ ou alkyle en C₁-C₆, lui-même éventuellement substitué par un ou plusieurs substituants choisis indépendamment parmi les atomes d'halogène et les groupements -NR¹⁴R¹⁵ et -OR¹⁶ ;
X représente un atome d'azote ou CH ;
Y représente un atome d'azote ou CH ;
R représente un atome d'hydrogène ou un groupement -NR¹⁷R¹⁸ ;
R¹⁷ et R¹⁸ représentent chacun indépendamment un atome d'hydrogène, ou un groupement 4-pipéridinyle, cycloalkyle en C₃-C₆ ou alkyle en C₁-C₈, dont les deux derniers groupements peuvent être éventuellement substitués par un ou plusieurs groupements substituants choisis indépendamment parmi les atomes d'halogène et les groupements - NR⁵R⁶, -CONR⁵R⁶, -OR⁹, -COOR⁷, -NR⁸COR⁹, -SR¹¹, -SO₂R¹¹, -SO₂NR⁵R⁶, -NR⁸SO₂R⁹, morpholinyle, alkyle en C₁-C₄, cycloalkyle en C₃-C₆, tétrahydrofuranyle, aryle et hétéroaryle, chacun des deux derniers groupements peut être éventuellement substitué par un ou plusieurs substituants choisis indépendamment parmi les atomes d'halogène ou les groupements cyano, nitro, -NR⁵R⁶, -CONR⁵R⁶, -OR⁹, -NR⁸COR⁹, -SO₂NR⁵R⁶, -NR⁸SO₂R⁹, alkyle en C₁-C₆ et trifluorométhyle, où R⁵, R⁶, R⁸ et R⁹ sont tels que définis ;
ou R¹⁷ et R¹⁸, pris ensemble avec l' atome d'azote auquel ils sont liés, forment un système de noyaux hétérocycliques saturé à 4 à 7 chaînons, lequel système de noyaux peut être éventuellement. substitué par un ou plusieurs groupements substituants choisis indépendamment parmi 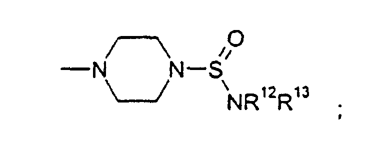 -NR⁵R⁶, -CONR⁵R⁶, -OR⁹, -COOR⁷, -NR⁸COR⁹, et alkyle en C₁-C₆ éventuellement substitué par un ou plusieurs substituants choisis indépendamment parmi les atomes d'halogène et les groupements -NR¹²R¹³ et -OR⁸,
R⁹ représente un atome d'hydrogène, un groupement alkyle en C₁-C₆ ou un groupement phényle, dont chacun peut être éventuellement substitué par un ou plusieurs groupements substituants choisis indépendamment parmi les atomes d'halogène, phényle, -OR¹⁶ et -NR¹⁴R¹⁵, et
chacun de R⁷, R⁸, R¹⁰, R¹¹, R¹², R¹³, R¹⁴, R¹⁵, et R¹⁶ représente indépendamment un atome d'hydrogène ou un groupement alkyle en C₁-C₆ ou phényle ;
• à condition que lorsque Y est CH, alors X soit N.

2. Composé selon la revendication 1, **caractérisé en ce que** A représente un groupement de formules (a) ou (c).

3. Composé selon la revendication 1 ou 2, **caractérisé en ce que** R¹ représente un groupement benzyle éventuellement substitué.

4. Composé selon l'une quelconque des revendications 1 à 3, **caractérisé en ce que** R² est CH(CH₃)CH₂OH, CH(Et)CH₂OH ou C(CH₃)₂CH₂OH.

5. Composé selon l'une quelconque des revendications 1 à 4, **caractérisé en ce que** R³ est hydrogène.

6. Composé selon la revendication 1, **caractérisé en ce qu'**il est choisi parmi :
le (2*R*)-2-[[2-amino-5-[(phénylméthyl)thio]thiazolo[5,4-*d*]pyrimidin-7-yl]amino]-1-propanol ;
la (1*R*)-5-[[Phénylméthyl]thio]-7-[(2-hydroxy-1-méthyléthyl)amino]thiazolo[5,4-*d*]pyrimidin-2(1*H*)-one ;
le (2*R*)-2-[2-[(Phénylméthyl)thio]-(1*H*-purin-6-yl)amino]-1-propanol ;
le (2*R*)-2-[[6-[[(2,3-Difluorophényl)méthyl]thio]-1*H*-pyrazolo[3,4-*d*]pyrimidin-4-yl]amino]-1-propanol et
le 2-Méthyl-2-[[5-[(phénylméthyl)thio]-3*H*-[1,2,3]-triazolo[4,5-*d*]pyrimidin-7-yl]amino]-1-propanol
et leurs sels et solvates pharmaceutiquement acceptables.

7. Procédé de préparation d'un composé de formule (I) tel que défini dans la revendication 1, **caractérisé en ce qu'**il comprend :
(1) pour des composés de formule (I) dans laquelle A est un groupement (a), le traitement d'un composé de formule (II) : 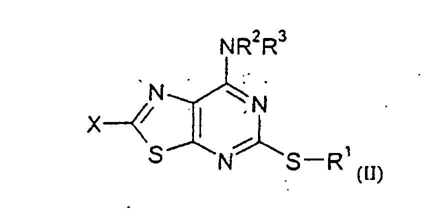 dans laquelle R¹, R² et R³ sont tels que définis dans la formule (I) ou en sont des dérivés protégés et X est un groupement partant, par un acide aqueux, ou
(2) pour les composés de formule (I) dans laquelle A est un groupement de formule (c), le traitement d'un composé de formule (IIA) : 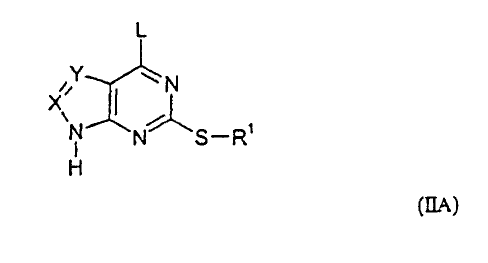 dans laquelle X, Y et R¹ sont tels que définis dans la formule (I) ou en sont des dérivés protégés et L est un groupement partant, par une amine HNR²R³, ou
(3) pour les composés de formule (I) dans laquelle A est un groupement de formule (c) et X et Y sont tous deux N, la réaction d'un composé de formule générale (IIB) : 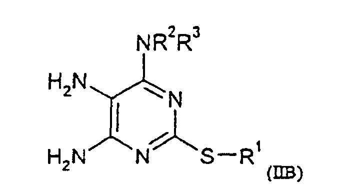 dans laquelle R¹, R² et R³ sont tels que définis dans la formule (I) ou en sont des dérivés protégés, avec un agent nitrosant, ou
(4) pour les composés de formule (I) dans laquelle A est un groupement (b) et où R¹, R² et R³ sont tels que définis dans la formule (I) ou en sont des dérivés protégés et R est NH₂, le traitement d'un composé de formule (III) par un agent réducteur : 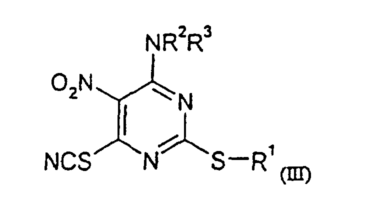 et éventuellement à la suite de l'un quelconque des procédés 1 à 4 :
• l'élimination de tout groupement protecteur présent
• la formation d'un sel ou d'un solvate pharmaceutiquement acceptable.

8. Composé intermédiaire de formule (IIB) tel que défini dans la revendication 7.

9. Composition pharmaceutique comprenant un composé de formule (I), ou un sel ou un solvate pharmaceutiquement acceptable de celui-ci, selon l'une quelconque des revendications 1 à 6 en association avec un adjuvant, un diluant ou un support pharmaceutiquement acceptable.

10. Procédé de préparation d'une composition pharmaceutique selon la revendication 9, **caractérisé en.ce qu'**il comprend le mélange d'un composé de formule (I), ou d'un sel ou d'un solvate pharmaceutiquement acceptable de celui-ci, selon l'une quelconque des revendications 1 à 6, avec un adjuvant, un diluant ou un support pharmaceutiquement acceptable.

11. Composé de formule (I), ou un sel ou un solvate pharmaceutiquement acceptable de celui-ci, selon l'une quelconque des revendications 1 à 6, destiné à être utilisé en thérapie.

12. Composé selon la revendication 11, destiné à être utilisé dans un procédé de traitement d'une maladie due à la médiation par les chémokines, **caractérisé en ce que** la chémokine se lie à un ou plusieurs récepteurs de la chémokine, comprenant l'administration à un patient d'une quantité thérapeutiquement efficace d'un composé de formule (I), ou d'un sel ou d'un solvate pharmaceutiquement acceptable de celui-ci, selon l'une quelconque des revendications 1 à 6.

13. Composé selon la revendication 12, **caractérisé en ce que** le récepteur de la chémokine appartient à la sous-famille des récepteurs de la chémokine CXC.

14. Composé selon la revendication 12 ou 13, **caractérisé en ce que** le récepteur de la chémokine est le récepteur CXCR2.

15. Composé selon la revendication 11, destiné à être utilisé dans un procédé de traitement d'une maladie inflammatoire chez un patient atteint, ou qui risque d'être atteint, de ladite maladie, comprenant l'administration au patient d'une quantité thérapeutiquement efficace d'un composé de formule (I), ou d'un sel ou d'un solvate pharmaceutiquement acceptable de celui-ci, selon l'une quelconque des revendications 1 à 6.

16. Composé selon la revendication 15, **caractérisé en ce que** la maladie est le psoriasis, une maladie dans laquelle l'angiogénèse est associée à des taux élevés de la chémokine CXCR2, ou la BPCO.

17. Composé selon la revendication 16, **caractérisé en ce que** la maladie est le psoriasis.

18. Composé, sel ou solvate selon la revendication 11, **caractérisé en ce que** la maladie est le cancer.

19. Composé, sel ou solvate selon la revendication 11, **caractérisé en ce qu'**il s'agit de maladie du tractus gastro-intestinal.

20. Composé, sel ou solvate selon la revendication 11, **caractérisé en ce que** la maladie est la BPCO.
